# EUROPEAN PATENT SPECIFICATION

(11) **EP 0 522 884 B1**
(45) Date of publication and mention of the grant of the patent: **29.10.1997**
(21) Application number: 92306396.0
(22) Date of filing: 13.07.1992
(51) Int. Cl.: C12Q 1/68

(54) **Process for controlling contamination of nucleic acid amplification reactions**
Verfahren zur Beherrschung der Verunreinigung von Nukleinsäure-Amplifizierungsreaktionen
Procédé pour contrôler la contamination des réactions d'amplification des acides nucléiques

(30) Priority: 12.07.1991 US 728874
(43) Date of publication of application: 13.01.1993
(73) Proprietor: Life Technologies, Inc., Rockville, MD 20850 (US)
(72) Inventor: Hartley, James L., Frederick, Maryland 21702 (US); Berninger, Mark, Gaithersburg, Maryland 20878 (US)
(74) Representative: Sheard, Andrew Gregory

(56) References cited:
- EP-A- 401 037
- EP-A- 415 755
- EP-A- 0 407 291
- Gene, 93, 1990, pages 125-128

## Description

The present invention relates to an improvement in processes which amplify nucleic acid sequences. In particular, the invention is directed to a means for eliminating the products of an execution of a nucleic acid amplification process that contaminate subsequent executions of the amplification process.

The invention seeks to provide a method for preventing carryover-contamination of nucleic acid samples by eliminating the products of an execution of an oligonucleotide-dependent nucleic acid amplification process that contaminate subsequent executions of the amplification process. Such oligonucleotide-dependent nucleic acid amplification processes may include, for example, Polymerase Chain Reaction (PCR) and Ligase Chain Reaction (LCR).

The present invention seeks to ensure that the results of an amplification process do not reflect the presence of a carryover-contaminating nucleic acid template.

The polymerase chain reaction (PCR) procedure amplifies specific nucleic acid sequences through a series of manipulations including denaturation, annealing of oligonucleotide primers, and extension of the primers with DNA polymerase (Mullis, K.B. *et al.,* US 4,683,202, US 4,683,195; Mullis, K.B., EP 201,184; Erlich, H., EP 50,424, EP 84,796, EP 258,017, EP 237,362; Erlich, H., US 4,582,788; Saiki, R. *et al.*, US 4,683,202; Mullis, KB. *et al.* Cold Spring Harbor *Symp. Quant. Biol. 51:263* (1986); Saiki, R. *et al. Science* 230:1350 (1985); Saiki, R. *et al. Science 231*:487 (1988); Loh, E.Y. *et al. Science 243:*217 (1988)). These steps can be repeated many times, potentially resulting in large amplification of the number of copies of the original specific sequence. It has been shown that even a single copy of a DNA sequence can be amplified to produce hundreds of nanograms of product (Li, H. *et al. Nature 335:*414 (1988)).

Other known nucleic acid amplification procedures include transcription-based amplification systems (Kwoh, D. *et al. Proc. Natl. Acad Sci. USA 86*:1173 (1989); Gingeras, T.R. *et al.,* WO 88/10315).

Schemes based on ligation of two (or more) oligonucleotides in the presence of a nucleic acid target having the sequence of the resulting "di-oligonucleotide," thereby amplifying the di-oligonucleotide, are also known (Wu, D.Y. and Wallace, R.B. *Genomics 4*:560 (1989); Backman *et al.*, EP 320,308; Wallace, B., EP 336,731; and Orgel, L., WO 89/09835). Such oligonucleotide-dependent amplifications are termed "Ligase Chain Reaction" (LCR).

A consequence of amplification processes, such as PCR or LCR, is that the amplification products themselves can be substrates for subsequent PCR or LCR procedures. Furthermore, because the quantities of the amplification products can be large, and because the sensitivity of PCR and LCR is so great, the dispersal of even an extremely small fraction of a reaction, such as a PCR or LCR reaction, into the laboratory area potentially can lead to contamination of later attempts to amplify other samples, thereby resulting in false positives. Extreme care must be taken to avoid carryover contamination (Kwok, S. and Higuchi, R. *Nature 339*:237 (1989)); this is very inconvenient and adds significantly to the cost of amplifications such as PCR and LCR.

Thus a need exists for a routine, economical method of nucleic acid amplification wherein such amplification may be performed without concern as to possible carryover-contamination from previous amplifications.

EP-A-0415755 and Gene, 93 (1990) 125-128 (Longo *et al)* disclose the use of primers in amplification processes, which primers include "exo-sample nucleotides" such as deoxyuridine. The primers disclosed are substantially susceptible to uracil DNA glycosylase.

The invention seeks to improve upon *in vitro* nucleic acid amplification procedures in general by making amplification products distinguishable from naturally occurring DNA. Accordingly, such products may be rendered inactive as templates for further amplification prior to the start of the succeeding amplification reaction.

This invention relates to a method of incorporating the nucleotide deoxyuridine (dU) into the amplified product strands resulting from a nucleic acid amplification process. Once the product strands have been obtained and analyzed (e.g. by hybridization, Southern blot, etc.), the sample strands can be selectively destroyed by acting on the incorporated deoxyuridine.

Two embodiments are presented. In a first embodiment, the deoxyuridine is incorporated by carrying out the amplification reaction in the presence of an excess of deoxyuridine tri-phosphate.

In a second embodiment, the deoxyuridine is incorporated by carrying out the amplification reaction in the presence of an oligonucleotide which has, as part of its sequence, one or more deoxyuridine nucleotides. The primer containing deoxyuridine nucleotide(s) can be used alone or in combination with the first embodiment, i.e., also incorporating the deoxyuridine by carrying out the amplification reaction in the presence of an excess of deoxyuridine tri-phosphate.

In a variation of the second embodiment, the deoxyuridine is incorporated in at least one oligonucleotide before the amplification reaction. The deoxyuridine is incorporated at the oligonucleotide termini. Before amplification, the oligonucleotide containing deoxyuridine is substantially amplifiable. After amplification, the amplified oligonucleotide (containing deoxyuridine) may be substantially unamplifiable. The oligonucleotide containing a deoxyuridine nucleotide may be made unamplifiable by a treatment during the amplification process. Causing the amplified oligonucleotide containing a deoxyuridine nucleotide to be cleaved at or near the location of the deoxyuridine is one example of making such an oligonucleotide substantially unamplifiable.

The invention may elimate the products of previous amplifications from further amplification by means of a treatment that leaves nucleic acid from the sample unaffected in its ability to be amplified. This treatment greatly reduces a major problem associated with amplification of nucleic acids, namely contamination of starting materials with the end products of previous amplification processes. In other words, this invention provides a process of discriminating against amplification products, and in favor of nucleic acids normally found in nature, prior to the start of succeeding amplification reactions.

More specifically, this invention relates to *in vitro* procedures which utilize enzymes to amplify specific nucleic acid sequences. Examples of such procedures include polymerase chain reaction (PCR) and ligase chain reaction (LCR). A serious limitation of the PCR procedure, the LCR procedure and other similar procedures is contamination of the laboratory environment with the amplified nucleic acid end products of individual reactions. Such contamination commonly results in amplification not only of authentic nucleic acid which may be present in the sample of interest, but also of the contaminating end products from previous reactions. This invention provides a process to remove possible contamination of this type, without affecting the desired amplification of authentic nucleic acids.

The first embodiment involves first performing amplification procedures in which the ribonucleoside triphosphate rTTP or deoxyribonucleoside triphosphate dTTP is replaced with rUTP or dUTP (normally absent from, or present very rarely in, nucleic acids found in the samples whose amplification is desired). The DNA or RNA produced during such amplification processes can be differentiated from sample nucleic acids. Thus, one can discriminate against nucleic acids produced during amplification processes in favor of sample DNA or RNA prior to or during succeeding amplification processes, such that previously amplified nucleic acid can no longer be amplified, while sample DNA or RNA remains amplifiable.

The present invention may represent an improvement upon *in vitro* oligonucleotide-dependent, nucleic acid amplification procedures. In the methods of the present invention, amplification products are made distinguishable from the nucleic acid substrate used to initiate the amplification in a manner which imparts distinct properties to the amplification products. Accordingly, prior to the start of a new amplification reaction, these distinct properties may be exploited so as to render former amplification products inactive as templates in subsequent amplification reactions.

Therefore, the present invention is directed to a process of oligonucleotide-dependent amplification of one or more nucleic acid sequences in a sample, the process comprising:
(a) amplifying nucleic acid present in a first sample to produce amplified first nucleic acid containing deoxyuridine;
(b) subjecting a second sample to uracil DNA glycosylase, which sample may be contaminated by the amplification product of (a); wherein the second sample contains a second nucleic acid and one or more oligonucleotides specific for use in amplifying said second nucleic acid, at least one of the olignucleotides comprising deoxyuridine at one or more of its termini and;
(c) amplifying the second nucleic acid;
   said at least one of the oligonucleotides being substantially less susceptible to uracil DNA glycosylase treatment than the amplified first nucleic acids containing deoxyuridine, so that uracil containing amplification products of (a) which may contaminate the second sample are rendered unamplifiable.

The process may further comprise the step of terminating the treatment of (b). The terminating step may be by heating.

The process as described above may be such that the oligonucleotide-dependent amplification is of two separate complementary strands, of equal or unequal length, and whereby amplifying the second nucleic acid (c) comprises the following steps:
(e) treating the complementary strands with two oligonucleotide primer, for each different specific sequence being amplified, under conditions such that for each different sequence being amplified, an extension product of each primer is synthesized which is complementary to each nucleic acid strand, the primers being sufficiently complementary to different strands of each specific sequence to hybridize therewith so that the extension product synthesized from one primer, when it is separated from its complement, can serve as a template for synthesis of the extension product of the other primer;
(f) separating the primer extension products from the templates on which they were synthesized to produce single-stranded molecules; and
(g) treating the single-stranded molecules generated from step (f) with the primers of step (e) under conditions that a primer extension product is synthesized using each of the single strands produced in step (f) as a template.

In addition, the present invention is directed to a method of detecting target nucleic acid in a sample, the method comprising:
(a) providing nucleic acid in the sample as single-stranded nucleic acid:
(b) providing the sample with at least four DNA probes, wherein:
   (1) the first probe is a single strand capable of hybridising to a first segment of a primary strand of the target nucleic acid;
   (2) the second probe is a single strand capable of hybridising to a second segment of the primary strand of the target nucleic acid sequence:
   (3) the 5' end of the first segment of the primary strand of the target is positioned relative to the 3' end of the second segment of the primary strand of the target to enable joining of the 3' end of the first probe to the 5' end of the second probe, when the probes are hybridised to the primary strand of the target nucleic acid;
   (4) the third probe is capable of hybridising to the first probe;
   (5) the fourth probe is capable of hybridising to the second probe;
   (6) at least the 3' nucleotide of the first probe or the 5' nucleotide of the second probe is deoxyuridine; and
   (7) at least the 3' nucleotide of the fourth probe or the 5' nucleotide of the third probe is deoxyuridine; and
(c) treating the sample with uracil DNA glycosylase; so that glycosidic bonds between the uracil and deoxyribose moieties of deoxyuridine of unjoined probes are substantially unaffected and glycosidic bonds between the uracil and deoxyribose moieties of deoxyuridine of joined probes are substantially cleaved, thereby forming an apyrimidinic deoxyribose in a sugar-phosphate backbone of a nucleic acid;
(d) repeatedly performing the cycle as follows:-
   (1) hybridising the probes with nucleic acid in the sample;
   (2) ligating hybridised probes to form joined probe sequences; and
   (3) denaturing DNA in the sample; and
(e) detecting the joined probe sequences.

The first and second probes described above may be designated "primary probes" which hybridise to first and second segments respectively of the primary strand of the target nucleic acid. The third and fourth probes described above may be designated "secondary nucleic acid probes" which hybridise to the first and second probes respectively.

Preferably, in step (6) above, both the 3' nucleotide of the first probe and the 5' nucleotide of the second probe are deoxyuridine; in step (7) above both the 3' nucleotide of the fourth probe and the 5' nucleotide of the third probe are deoxyuridine; and/or; in (6) and (7) above both the 3' nucleotide of the first probe and the 3' nucleotide of the fourth probe are deoxyuridine.

The method of detecting target nucleic acid may additionally comprise the steps of (f) treating the sample with endonuclease IV, and/or subjecting the sample to alkaline hydrolysis to cleave a sugar-phosphate backbone; and (g) optionally terminating the treatment such as by heating.

The method may be carried out such that the probes are joined by ligase; the target nucleic acid is DNA; the joined nucleic acid is separated from the target sequence by heat denaturation, and/or the cycle is repeated at least twice, such as between 20 and 50 times.

The method may be carried out wherein the 5' end of the second probe, but not of the first probe is phosphorylated and/or the target sequence is double stranded before step (a).

In the description that follows, a number of terms used in molecular biology and nucleic acid amplification technology are extensively utilized. In order to provide a clearer consistent understanding of the specification and claims, including the scope to be given such terms, the following definitions are provided.

"Amplification", as used herein, refers to any *in vitro* process for increasing the number of copies of a nucleotide sequence or sequences. Nucleic acid amplification results in the incorporation of nucleotides into DNA or RNA. As used herein, one amplification reaction may consist of many rounds of DNA replication. For example, one PCR reaction may consist of 30-100 "cycles" of denaturation and replication.

"Nucleotide" as used herein, is a term of art that refers to a base-sugar-phosphate combination. Nucleotides are the monomeric units of nucleic acid polymers, i.e. of DNA and RNA. The term includes ribonucleoside triphosphates, such as rATP, rCPT, rGTP, or rUTP, and deoxyribonucleotide triphosphates, such as dATP, dCTP, dUTP, dGTP, or dTTP. A "nucleoside" is a base-sugar combination i.e. a nucleotide lacking phosphate. It is recognized in the art that there is a certain interchangeability in usage of the terms nucleoside and nucleotide. For example, the nucleotide deoxyuridine triphosphate, dUTP, is a deoxyribonucleoside triphosphate. After incorporation into DNA, it serves as a DNA monomer, formally being deoxyuridylate, i.e. dUMP or deoxyuridine monophosphate. One may say that one incorporates dUTP into DNA even though there is no dUTP moiety in the resultant DNA. Similarly, one may say that one incorporates deoxyuridine into DNA even though that is only a part of the substrate molecule.

Deoxyuridine is an example of a nucleotide which as used herein, refers to a nucleotide which is generally not found in the sample or sequence to be amplified. Although the triphosphate form of deoxyuridine, dUTP, is present in living organisms as a metabolic intermediate, it is rarely incorporated into DNA. When dUTP is incorporated into DNA, the resulting deoxyuridine is promptly removed *in vivo* by normal processes, e.g. processes involving the enzyme uracil DNA glycosylase (UDG). Thus, deoxyuridine occurs rarely or never in natural DNA. The presence of deoxyuridine may be determined readily using methods well known to the art. Numerous DNA glycosylases are known to the art.

"Uracil DNA glycosylase" (UDG), a term of art, refers to an enzyme which cleaves the glycosidic bond between the base uracil and the sugar deoxyribose, only when the monomeric nucleotide dUTP is incorporated into a DNA molecule, resulting in incorporation of a deoxyuridine moiety (Duncan, B. in *The Enzymes 14:*565 (1981), ed.: Boyer P). The enzyme does not act upon free dUTP, free deoxyuridine, or RNA (Duncan, *supra).*

"Incorporating" as used herein, means becoming part of a nucleic acid polymer.

"Terminating" as used herein, means causing a treatment to stop. The term includes means for both permanent and conditional stoppages. For example, if the treatment is enzymatic, a permanent stoppage would be heat denaturation; a conditional stoppage would be, for example, use of a temperature outside the enzyme's active range. Both types of termination are intended to fall within the scope of this term.

"Oligonucleotide" as used herein refers collectively and interchangeably to two terms of art, "oligonucleotide" and "polynucleotide". Note that although oligonucleotide and polynucleotide are distinct terms of art, there is no exact dividing line between them and they are used interchangeably herein. The term "probe" may also be used interchangeably with the terms "oligonucleotide" and "polynucleotide".

"Oligonucleotide-dependent amplification" as used herein refers to amplification using an oligonucleotide, polynucleotide or a probe to amplify a nucleic acid sequence. An oligonucleotide-dependent amplification is any amplification that requires the presence of one or more oligonucleotides, polynucleotides or probes that are two or more mononucleotide subunits in length and that end up as part of the newly-formed, amplified nucleic acid molecule.

"Primer" as used herein refers to a single-stranded oligonucleotide or a single-stranded polynucleotide that is extended by covalent addition of nucleotide monomers during amplification. Nucleic acid amplification often is based on nucleic acid synthesis by a nucleic acid polymerase. Many such polymerases require the presence of a primer that can be extended to initiate such nucleic acid synthesis.

"Substantially unamplifiable" as used herein refers to the inhibition of amplification of nucleic acids in any *in vitro* process. When the nucleic acid is said to be "substantially unamplifiable", the majority of the nucleic acid molecules (≥ 50%) cannot be or are inhibited from being amplified, for example, by PCR or LCR. Although the term "substantially unamplifiable" indicates that the amplification of nucleic acid sequences is prevented or inhibited from being amplified, some amplification may still occur but such "background" amplification of a "substantially unamplifiable" nucleic acid can be distinguished from amplification of other nucleic acids which are "substantially amplifiable." Nucleic acids which are "substantially amplifiable" are, relatively speaking, amplified to a greater extent (e.g. at least two fold or more) than "substantially unamplifiable" nucleic acids. One of ordinary skill in the art may easily utilize controls to distinguish between amplification of "substantially unamplifiable" nucleic acids and "substantially amplifiable" nucleic acids in accordance with the present invention.

In the process according to the first embodiment, an amplification procedure is performed on a first sample in which one or more of rTTP or dTTP is replaced with rUTP or dUTP. Before a second amplification, any contaminating amplified product that may be remaining is subjected to a treatment which renders nucleic acid containing the deoxyuridine substantially unamplifiable. The treatment is done in the presence of a second sample containing nucleic acid sequences to be amplified. The amplified nucleic acid sequences derived from the first sample which contaminate the second sample are not further substantially amplified during amplification of nucleic acid sequences of the second sample.

Deoxyribonucleoside triphosphate dUTP is a nucleotide which may be conveniently incorporated into an enzymatic DNA amplification procedure, exemplified herein by PCR, thereby resulting in deoxyuridine-containing DNA. The DNA products of such a reaction will normally contain many uracil bases. Discrimination between natural DNA and the resultant, deoxyuridine-containing products of amplification procedures may be obtained with the enzyme uracil DNA glycosylase (UDG). Treatment of DNA containing uracil bases with uracil DNA glycosylase results in cleavage of the glycosidic bond between the deoxyribose of the DNA sugar-phosphate backbone and the uracil base. The loss of the uracil creates an apyrimidinic site in the DNA, which blocks DNA polymerase from using the DNA strand as a template for the synthesis of a complementary DNA strand (Schaaper, R., *et al., Proc. Natl. Acad. Sci. USA 80:*487 (1983)). The presence of substantial numbers of apyrimidinic sites in each DNA target molecule interferes with amplification procedures which use DNA polymerase to synthesize copies of target DNA. The DNA sugar-phosphate backbone that remains after UDG cleavage of the glycosidic bond can then be cleaved by endonuclease IV, alkaline hydrolysis, tripeptides containing aromatic residues between basic ones such as Lys-Trp-Lys and Lys-Tyr-Lys (Pierre *et al, J. Biol. Chem. 256:*10217-10226 (1981)) and the like.

As exemplified herein, the basic amplification protocol is the well known PCR method. PCR was modified in three ways: (1) dUTP was substituted for dTTP; (2) UDG was added to the initial PCR reaction mixture; and (3) an initial incubation period was added to allow UDG to destroy contaminating products of prior PCR reactions. The UDG itself was either permanently inactivated by high temperature in the first PCR cycle or was not active at the high temperatures used with Taq polymerase in the currently preferred PCR protocol. This inactivation prevents UDG from destroying newly-synthesized PCR products. Nucleic acid amplification protocols that do not eliminate UDG activity usually will require an extra UDG-inactivation step.

The methods of the invention may allow amplification products to be distinguished from the original nucleic acid substrate in a manner which does not interfere with detection analysis of such amplification products but which may make such amplification products uniquely susceptible to certain treatments which destroy their ability to serve as templates in subsequent amplification reactions. Due to this susceptibility, pretreating samples to be amplified according to the methods of this embodiment may allow the elimination of any contaminating products of previous amplifications from the sample. In the first embodiment, deoxyuridine is incorporated into amplification products during the amplification procedure itself so as to distinguish such amplified sequences from the original template.

In the second embodiment, the deoxyuridine used to distinguish such amplification products herein is provided as part of an oligomer or polymer before amplification, while that of the first embodiment is provided as a deoxyuridine triphosphate which becomes incorporated into polymeric nucleic acid during amplification.

The methods of the second embodiment are applicable to any *in vitro* procedures which utilize enzymes to amplify specific nucleic acid sequences and especially to PCR and LCR.

In a preferred variation of the second embodiment, the nucleic acid products produced by an amplification which uses primers containing deoxyuridine are chemically different from starting templates which were not produced by an amplification containing deoxyuridine. This chemical difference permits one to render nucleic acid products, which were produced by an amplification which used primers containing deoxyuridine, incapable of further exponential amplification.

Incorporation of an deoxyuridine into a primer or probe allows the DNA or RNA produced during such amplification processes to be differentiated from the original nucleic acids present in the sample prior to amplification. If desired, the amplification reaction itself may, in addition, further provide deoxyuridine for incorporation into the replicating nucleic acid.

Embodiments utilizing oligonucleotides with high proportions of deoxyuridine are preferred over those with fewer deoxyuridine containing oligonucleotides. Especially, oligonucleotides with a high fraction of deoxyuridine located at the 3'OH end of the oligonucleotide are preferred. In another preferred embodiment, a deoxyuridine nucleotide is the 3' nucleotide.

In one variation of the second embodiment, treatment of deoxyuridine containing primers to render amplified nucleic acid unamplifiable is performed before beginning an amplification reaction.

In another variation of the second embodiment, treatment to render amplified nucleic acid unamplifiable is performed after terminating a first amplification reaction and after removing a sample of the amplified products for further analysis, but before subjecting the original, starting nucleic acid in the sample to a new, second amplification reaction. For example, after amplification and after removing amplified samples for analysis, amplified product that may be remaining is subjected to uracil DNA glycosylase treatment which renders amplified nucleic acid containing the deoxyuridine substantially unamplifiable.

Treatment to render the amplified nucleic acid containing the deoxyuridine unamplifiable is by uracil DNA glycosylase treatment. Such treatment may be done in the presence of a second sample containing nucleic acid sequences to be amplified. Accordingly, amplified nucleic acid sequences produced by amplification of a first sample in the presence of deoxyuridine nucleotides which contaminate a second sample may not be further substantially amplified during the second oligonucleotide-dependent amplification of nucleic acid sequences even if the same oligonucleotide(s) are used as primers or probes.

Discrimination between a nucleic acid which does not contain the nucleotide deoxyuridine and a deoxyuridine-containing product of an amplification reaction is obtained with the enzyme UDG. Treatment of DNA containing uracil bases with UDG results in cleavage of the glycosidic bond between the deoxyribose of the DNA sugar-phosphate backbone and the uracil base. The loss of the uracil creates an apyrimidinic site in the DNA, which blocks DNA polymerase from using the DNA strand as a template for the synthesis of a complementary DNA strand (Schaaper, R. *et al. Proc. Natl. Acad. Sci. USA 80:*487 (1983)). The presence of substantial numbers of apyrimidinic sites in each DNA target molecule interferes with amplification procedures which use DNA polymerase to synthesize copies of target DNA. The DNA sugar-phosphate backbone that remains after UDG cleavage of the glycosidic bond can then be cleaved by endonuclease IV, alkaline hydrolysis, tripeptides containing aromatic residues between basic ones such as Lys-Trp-Lys and Lys-Tyr-Lys (Pierre *et al., J. Biol. Chem. 256*:10217-10226 (1981) and Doetsch *et al. Mutation Research 236*:173-201 (1990)) and the like.

By providing primers containing deoxyuridine, such deoxyuridine nucleotides are localized at the 5' ends of each strand of DNA template which is amplified. When deoxyuridine-containing primers have been used and the sample treated with UDG, substantial numbers of apyrimidinic sites in the 5'-end of each DNA target template molecule are found. Such apyrimidinic sites interfere with extension at the 3'-end of newly made strands. These 3'-end sequences are the targets to which original deoxyuridine sample-containing primers bind. Thus these primers do have target sequences with which they can bind a nucleic acid derived from molecules primed by deoxyuridine sample nucleotide-containing primers.

Oligonucleotides that contain deoxyuridine at their termini are not substantially susceptible to UDG cleavage of the glycosidic bond between the deoxyribose of the DNA sugar-phosphate backbone and the uracil base. Thus, particularly in LCR reactions, such oligonucleotides containing deoxyuridine at their termini are amplifiable even after treatment with UDG.

After amplification, preferably by LCR, the joined or ligated oligonucleotides (probes) containing deoxyuridine are substantially cleaved by UDG, thereby forming an apyrimidinic deoxyribose in a sugar-phosphate backbone of the ligated or joined oligonucleotides. The resulting sugar-phosphate backbone can be cleaved by treatment with endonuclease IV, alkaline hydrolysis, tripeptides containing aromatic residues between basic ones (Pierre et *al., J. Biol. Chem. 256*:10271-10220 (1991)) and the like. Thus, UDG treatment, and optionally the cleavage of the sugar-phosphate backbone, substantially prevents amplification of the ligation product, thereby allowing one to distinguish and eliminate such amplification products as possible contaminants from subsequent amplification reactions.

As exemplified herein, in a preferred variation of the second embodiment, the basic amplification protocol PCR is modified in three ways: (1) amplification is first performed using oligonucleotide primers containing deoxyuracil substituted for deoxythymidine and samples of the amplified products are removed for analysis; (2) UDG is then added to subsequent PCR reaction mixtures; and (3) an incubation period is added to allow UDG to act on uracil-containing sequences in contaminating products from prior PCR reactions.

As exemplified herein, in another preferred variation of the second embodiment, the basic amplification protocol LCR is modified in three ways: (1) amplification is first performed using oligonucleotide probes containing deoxyuracil substituted for deoxythymidine at one or more terminus of the oligonucleotides and samples of the amplified products are removed for analysis; (2) UDG is then added to subsequent LCR reaction mixtures; and (3) an incubation period is added to allow UDG to act on uracil-containing sequences in contaminating products from prior LCR reactions. The UDG treatment alone has been found to substantially prevent amplification of the uracil-containing ligation product without substantially affecting amplification of the oligonucleotide probes containing deoxyuracil near the end of the probes. As an alternative to UDG treatment alone, UDG and a treatment that cleaves the sugar-phosphate backbone (remaining after UDG treatment) may be used. Such treatments that cleave the sugar-phosphate backbone may include, but are not limited to, endonuclease IV, alkaline hydrolysis, tripeptides such as Lys-Trp-Lys and Lys-Tyr-Lys (Pierre *et al., J. Biol. Chem. 256*:10217-10220 (1981)), AP endonucleases such as endonuclease V, endonuclease III, endonuclease VI, endonuclease VII, human endonuclease II, and the like (Linn, S. *Nucleases Involved in DNA Repair* in *Nucleases,* Cold Spring Harbor Laboratory, ed. Linn, S. and Roberts, R. (1985)).

Before continuing with the amplification procedure, the UDG and the enzyme treatment to cleave the sugar-phosphate backbone (if present) should be inactivated. UDG and the enzyme treatment to cleave the sugar-phosphate backbone may be either permanently inactivated by high temperature in the first PCR or LCR cycle, or by the high temperatures used with Taq DNA polymerase or Taq DNA ligase in the currently preferred PCR or LCR protocols, respectively. This inactivation prevents UDG from destroying newly-synthesized PCR or LCR products. Nucleic acid amplification protocols that do not inherently eliminate UDG activity usually will require an extra UDG-inactivation step.

Embodiments having a greater proportion of oligonucleotides containing deoxyuridine are preferred. However, even in standard PCR embodiments that depend on two oligonucleotide primers, the present invention is capable of rendering PCR contaminants unamplifiable as long as at least one primer contains deoxyuridine.

Not all single deoxyuridine containing oligonucleotides may reduce amplification of PCR or LCR products to sufficiently low levels for a given situation; those of ordinary skill in the art can empirically find which primers or probes are acceptable during routine optimization and testing of a PCR or LCR assay, without undue experimentation.

Routine assay optimization, aimed at testing oligonucleotide suitability, can be done by (1) making a deoxyuridine containing oligonucleotide, (2) performing a first nucleic acid amplification of a target sequence using that oligonucleotide, (3) seeding various amounts of the resulting first product in a new, second amplification that does not contain target sequences, (4) treating the second amplification to render deoxyuridine containing nucleic acid unamplifiable, (5) performing the second amplification, and (6) assaying the resultant second product for presence of contaminating sequence from the first hybridization. All of these steps are routinely done as experimental controls and as part of the normal validation of a method. The only additional work required of one practicing this embodiment involves routine synthesis of any additional oligonucleotides that may be required. Generally, if a oligonucleotide is observed to be not suitable, a substitute can be easily found and similarly tested.

As will be understood by those of ordinary skill in the art, the methods of the invention prevent exponential amplification but may not prevent linear amplification. Linear amplification does not usually represent a substantial problem in amplification procedures. For example, a single amplification product molecule which contaminates a sample run through 20 cycles of PCR, amplifying by a factor of two on each cycle, will result in only about 20 molecules if amplified linearly but could result in up to about a million molecules if amplified exponentially at maximal theoretical efficiency. Thus, linear amplification is generally inconsequential.

The second embodiment can be adapted to be used with amplifications that do not use a nucleic acid polymerase. For instance, as exemplified herein, contamination of samples with previously made products can be controlled by amplification schemes that polymerize oligonucleotides with ligase (e.g. Wu and Wallace, *supra,* and Barany, *Proc. Natl. Acad. Sci. USA 88:*189-193 (1991)).

The present invention is also ideally suited for and provides a kit for performing an assay for the method of detecting target nucleic acid as described above, according to the invention. The kit comprises a container comprising the probes, a container comprising a nucleic acid ligase, a container comprising a ligase, a container comprising a uracil DNA glycosylase and optionally a container comprising endonuclease IV.

Such a kit may comprise a carrier which is compartmentalized to receive in close confinement the containers such as vials, test tubes, bottles and the like. As will be evident, the components included in the kit such as nucleic acid ligase, a uracil DNA glycosylase etc. may be mixed in various combinations and such combinations may be included in a single or multiple container means.

The invention will now be described with reference to the accompanying drawing and Examples which are included for purposes of illustration only and are not to be construed as being limiting. In the drawing:

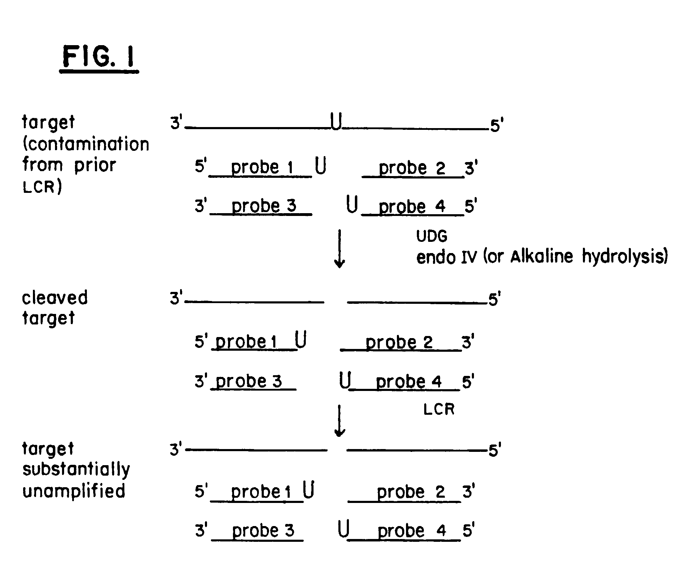

Figure 1 is a schematic diagram of the application of deoxyuridine nucleotides in ligase chain reaction.

### EXAMPLE 1

A polymerase chain reaction (PCR) was performed to amplify a region of the human papilloma virus type 16 (HPV 16) DNA (Durst, M. *et al., Proc. Natl. Acad Sci. USA 80:*3812 (1983)). The sequences of the primers used were 5'GGTCGATGTATGTCTTGTTG3' and 5'GTCTACGTGTGTGCTTTGTAC3'.

HPV 16 DNA was excised from a full length plasmid clone, pT7HPV16 (for the purposes of this invention, equivalent to the pUC8 plasmids descnbed by Seedoff, K, *et al., Virol.* 145:181 (1985)) with the restriction enzyme BamH I. The linear DNA (10 picograms) was added to PCR reactions containing 50 microliters of 25 mM Tris HCl pH 8.3, 5 mM MgCl₂, 50 mM NaCl, 0.01% gelatin, 0.05% W1 polyoxyethylene ether detergent (Sigma), 0.2 mM each dATP, dGTP, dCTP, 0.2 mM either dUTP or dTTP, 1 micromolar of each primer, and 2.5 units of thermostable DNA polymerase from *Thermus aquaticus* (Cetus/Perkin-Elmer). The reactions were amplified in a thermal cylcer (Cetus/Perkin-Elmer) using the following temperature profile: 5 minutes at 94°C, then 30 cycles of 1 minute at 94°C (denaturation), two minutes at 55°C (annealing), and 3 minutes at 72°C (primer extension). After completion of the temperature cycles, a final extension of 10 minutes at 72°C was done. Amplification of the 284 base pair HPV 16 DNA fragment was confirmed by agarose/ethidium bromide gel electrophoresis (Maniatis, T., *et al., Molecular Cloning,* Cold Spring Harbor Laboratory (1982)) of the PCR reaction products (5 microliters of each reaction per lane). All reactions showed substantial amplification. Negative control reactions to which no HPV 16 DNA was added did not produce any visible DNA products.

The concentration of the PCR amplification products was estimated from the agarose gel. New PCR reactions were contaminated with ten femtogram quantities of the amplification products that contained either deoxythymidine, resulting from incorporation of dTTP, or deoxyuridine, from dUTP-containing reactions. Positive control reactions contained 10 picograms of linear HPV 16 DNA. Negative control reactions did not receive any target DNA. The new PCR reactions contained dUTP instead of dTTP, and either 5 nanograms of UDG (Van de Sande, H., University of Calgary; also available from Life Technologies Inc., P.O. Box 9418, Gaithersburg, MD, 20898) or no UDG. All reactions were incubated for 15 minutes at 37°C to allow the UDG to act on deoxyuridine-containing DNA, and then were taken through the same thermal cycling protocol as above. Aliquots of each reaction were analyzed by agarose/ethidium bromide gel electrophoresis.

The agarose gel analysis showed that without UDG treatment the deoxyuridine-containing PCR products could be re-amplified to give a DNA product virtually indistinguishable in size, as evidenced by gel electrophoresis, from the products obtained by amplifying the normal HPV 16 DNA. Reactions in which the deoxyuridine-containing DNA was incubated with UDG prior to PCR did not give any visible products on the agarose gel. PCR amplification products that contained deoxythymidine were successfully amplified whether or not they had been incubated with UDG. This experiment showed that UDG substantially abolished amplification of PCR products containing deoxyuridine, but had no substantial effect on the amplification of DNA containing deoxythymidine.

Although the foregoing refers to particular preferred variations of the first embodiment, it will be understood that this embodiment is not so limited. It will occur to those of ordinary skill in the art that various modifications may be made to the disclosed embodiments and that such modifications are intended to be within the scope of this embodiment.

### EXAMPLE 2

### General Experimental Conditions of the Second Embodiment

Polymerase chain reactions (PCRs) were performed to amplify a region of the human papilloma virus type 16 (HPV 16) DNA (Durst, M. *et al. Proc. Natl. Acad. Sci. USA 80:*3812 (1983)). The sequences of the oligonucleotide primers used were either 5'GGUCGAUGUAUGUCUUGUUG3' and 5'GUCUACGUGUGUGCUUUGUAC3'(dU primers dU₁ and dU₂, respectively) or 5'GGTCGATGTATGTCTTGTTG3' and 5'GTCTACGTGTGTGCTTTGTAC3' (control dT primers dT₁ and dT₂, respectively). Note that the sequences of dT₁ and dU₁ were identical except for the replacement of U for T; dT₂ and dU₂ were similarly identical.

HPV 16 DNA was excised from a full length plasmid clone, pT7HPV16 (for the purposes of this invention, equivalent to the pUC8-based plasmids described by Seedoff, K. *et al. Virol. 145*:181 (1985)) with the restriction enzyme BamH I. The linear DNA (10 picograms) was added to PCR reactions containing 50 microliters of 25 mM Tris HCI pH 8.3, 5 mM MgCl₂, 50 mM NaCl, 0.01% gelatin, 0.2 mM each dATP, dGTP, dCTP, 0.2 mM either dUTP or dTTP, 1 micromolar of each primer, and 2.5 units of thermostable DNA polymerase from *Thermus* *aquaticus* (Cetus/Perkin-Elmer). Primers and triphosphates were paired as follows: dU primers with dTTP (pUxT reactions ("primed with U, extended with T")) and control dT primers with dUTP (pTxU reactions). The reactions were amplified in a thermal cylcer (Cetus/Perkin-Elmer) using the following temperature profile: 5 minutes at 94°C, then 30 cycles of 1 minute at 94°C (denaturation), two minutes at 55°C (annealing), and 3 minutes at 72°C (primer extension). After completion of the temperature cycles, a final extension of 10 minutes at 72°C was done. Amplification of the 284 base pair HPV 16 DNA fragment was confirmed by agarose/ethidium bromide gel electrophoresis (Maniatis, T., *et al.*, *Molecular Cloning,* Cold Spring Harbor Laboratory (1982)) of the PCR reaction products (5 microliters of each reaction per lane). All reactions showed substantial amplification. Negative control reactions to which no HPV 16 DNA was added did not produce any DNA products. The concentration of the PCR amplification products was estimated from the agarose gel.

### EXAMPLE 3

### Amplification Using Two Deoxyuridine-Containing Primers

New pUxT PCR reactions were prepared that contained dTTP but lacked dU primers. These were contaminated with ten femtograms of pUxT amplification products from the previous reactions, and incubated for 15 minutes at 37°C in the presence or absence of UDG (H. Van de Sande, University of Calgary; also available from Life Technologies Inc., P.O. Box 9418, Gaithersburg, MD 20898). These reactions were then incubated at 94°C to inactivate the UDG, cooled to 15°C, and dU primers were added.

New pTxU PCR reactions were prepared with dT primers and dUTP and were contaminated with ten femtograms of pTxU amplification products. These reactions were incubated at 37°C for 15 minutes in the presence or absence of UDG to act as controls for UDG activity and PCR amplification, respectively.

The new PCR reactions were then subjected to the same PCR temperature cycling described above. Agarose/ethidium bromide gel electrophoresis showed that in the absence of UDG treatment both pUxT and pTxU reaction products could be reamplified by PCR to give results identical in appearance to those obtained upon amplification of HPV 16 DNA. In contrast, UDG treatment abolished reamplification of both pTxU reaction products (the positive control) and pUxT reaction products, which contained dU primers. In another experiment it was shown that, in the absence of deoxyuridine in either the primers or extension products, UDG did not affect the PCR amplification of natural HPV 16 DNA.

Without UDG treatment, the deoxyuridine-containing PCR products could be re-amplified to give a DNA product virtually indistinguishable in size, as evidenced by agarose gel electrophoresis, from the products which did not contain deoxyuridine and which were obtained by amplifying the normal HPV 16 DNA. The reaction in which the DNA was made with deoxyuridine-containing primers and which was incubated with UDG prior to PCR did not substantially give any visible products on the agarose gel. In another experiment it was shown that UDG did not affect the amplification of natural DNA.

### EXAMPLE 4

### Amplification Using a Single Deoxyuridine-Containing Primer

A further experiment demonstrated that only one of two PCR primers need contain an exo-sample nucleotide. PCR amplifications were done essentially as described above using the following pairs of primers: (1) dT₁ with dT₂, (2) dT₁ with dU₂, (3) dU₁ with dT₂, and (4) dU₁ with dU₂. All combinations of PCR products were treated both with and without UDG before the second round of PCR. Amplification (1) tested for success of PCR amplification while amplification (4) tested for activity of UDG and the ability to eliminate contamination. Amplification (3) was observed to not be substantially reamplified after UDG treatment in a second round of PCR, thereby demonstrating that only one of a pair of PCR primers need contain a deoxyuridine nucleotide in order to reduce or eliminate the effects of sample contamination. Amplification (2) was observed to be reamplified, though at a substantially lower level than amplification (1).

To summarise, PCR amplification products that contained deoxyuridine in a primer or primers were successfully amplified if no UDG was present; amplification could be prevented by prior incubation with UDG. In other words, UDG could substantially abolish amplification of PCR products made with deoxyuridine-containing primers but had no substantial effect on the amplification of DNA made with deoxythymidine-containing primers.

### EXAMPLE 5

A modification of the Ligase Chain Reaction (LCR) described by Barany (F. Barany, *Proc. Natl. Acad. Sci. 88,* 189-193, 1991) was performed to amplify a region of the human beta globin gene. A schematic of this experiment demonstrating the application of deoxyuridine nucleotides in LCR is shown in figure 1.

The sequences of the probe oligonucleotides were: # 1088 (probe 4): 5′ acc atg gtg cac ctg act ccu 3′ 
# 1089 (probe 3): 5′ gag gag aag tct gcc gtt act 3′
# 1090 (probe 2): 5′ cag gag tca ggt gca cca tg 3′
# 1091 (probe 1): 5′ cag taa cgg cag act tct ccu 3′
 The sequence of the target oligonucleotide was:
# 1092 (target): 5' gac acc atg gtg cac ctg act ccu gag gag aag tct gcc gtt act gcc ctg 3'
Oligonucleotides were chemically synthesized by standard techniques. They were trace labeled with ³²P (approximately 30 dpm/pmol each oligonucleotide) and gel purified, and their quantities were estimated by hybridization and electrophoresis on 5% agarose/ethidium bromide gels. Thermostable DNA ligase from *Thermus aquaticus* was purchased from Molecular Biology Resources (MBR), Milwaukee, WI. UDG was from Life Technologies, Inc., Gaithersburg, MD. The buffer used was that suggested by MBR, and contained 20 mM Tris HCl pH 7.5, 25 mM KCl, 10 mM MgCl₂, 10 mM dithiothreitol, 0.6 mM NAD, 0.1% Triton® X100. Probe oligonucleotide #1089 was labeled at its 5' terminus with ³²P using T4 polynucleotide kinase to a specific activity of about 250,000 dpm per picomole, then chased with unlabelled rATP and the kinase was heat inactivated. The final concentration of the labelled #1089 oligonucleotide was about 1 picomole per microliter. Oligonucleotide #1090 was phosphorylated at its 5' end with rATP and T4 polynucleotide kinase.

Reactions for UDG treatment contained 20 pmol each of all four trace labeled probe oligos with or without 2 pmol target These samples were incubated in 10 ul 50 mM Tris HCl pH 8.0, 1 mM EDTA under oil for 15 min at 37° in the presence or absence of 0.5 µl = 0.5 units UDG, as follows:
A: Probes only, no UDG
B: Probes only, + UDG
C: Probes + target, no UDG (should support LCR)
D: Probes + target + UDG (target degraded, no LCR)
E: Probes only, + UDG

Any abasic sites (apyrimidinic sites) produced by UDG were broken (i.e., cleavage of the sugar phosphate backbone) by alkaline hydrolysis as follows: to all five tubes was added 5 µl 0.2 M NaOH giving a pH of about 13. The tubes were incubated at 95° for 10 minutes, cooled, and 5 µl of 2.5% acetic acid were added to bring the pH back to about 5. Any target (oligo # 1092) present in the reactions was cleaved by the combination of base removal by UDG and breakage of the sugar-phosphate backbone by elevated temperature and pH.

At this time (after UDG inactivation by alkaline pH and heat) 1 ul (2 pmol) target (#1092) was added to reaction E. Thus the target in reaction "E" was never exposed to active UDG, but only to inactivated UDG.

The concentrations of the salt produced by the alkaline hydrolysis reactions were reduced to avoid interference with subsequent ligase reactions and gel electrophoresis. Five µl of each reaction were diluted 1:40 in 1X MBR buffer (20 mM Tris HCI pH 7.5, 25 mM KCI, 10 mM MgCl₂, 10 mM dithiothreitol, 0.6 mM NAD, 0.1% Triton® X100). To 20 µl each diluted oligo mixture was added 0.5 ul = 500 fmol ³²P-labelled #1089 to follow the progress of the LCR reactions. Four µl of these mixtures containing 100 fmol probes 5'OH-1088, 5'P-1090, and 5'-OH-1091, 200 fmol 5'-³²P 1089, with or without 10 fmol 5'OH-target #1092, were aliquotted to pairs of tubes and 16 µl of lx MBR buffer with ("+") or without ("-") 10 units of MBR Taq ligase were added. A drop of oil was added to the 20 ul reactions and they were cycled 85° 30s, 50° 2 min, 20 times in a Cetus/Perkin Elmer Thermal Cycler. 2.5 µl each reaction were applied to a 12% denaturing sequencing gel using standard techniques (Maniatis, *supra).*

Following electrophoresis and exposure of the gel to X-ray film, the following results in the presence of ligase were observed:

The "A" reaction (probes only, no UDG treatment) showed a minimal amount (about 5% of the starting probes) of target-independent amplification (i.e., ligation product of about 40 nucleotides was produced).

The "B" reaction (probes treated with UDG) showed minimal target-independent amplification indistinguishable from reaction "A". This indicated that UDG had no effect on the integrity of the probes.

The "C" reaction (containing probes and target without UDG treatment) showed the majority (about 70%) of the probes were converted to product by target-dependent ligation.

Reaction "D" (containing probes and target treated with UDG) showed substantial decrease in the amount of ligation product present. This was the expected result if the uracil in the target had been removed by UDG and the sugar-phosphate backbone had been broken by elevated pH and temperature treatment (Figure 1).

Reaction "E" (probes treated with UDG, and target added after UDG inactivation by pH and heat) showed the majority of the probes (about 70%) had been ligated to form product This result demonstrated that the probes had not been affected by the UDG treatment, allowing them to participate in the ligation reactions.

The amounts of product produced in reactions C, D, and E show that UDG is active on a uracil positioned in the sequence of target oligonucleotide (i.e. not at the terminus), but is inactive on uracils at the 3' termini of oligonucleotides. It has also been demonstrated that UDG had relatively little activity on uracils located at the 5' terminus of an oligonucleotide.

To confirm the presence of uracil at the 3' terminus of probe # 1088, products of reactions C and E were pooled, precipitated with ethanol, and dissolved in 20 µl of water. One ul of 1M Tris HCI pH 8.0 and one µl of 20 mM EDTA were added to the pooled oligonucleotides and two 5 µl aliquots were placed in tubes. To one tube, 0.5µl (0.5 units) BRL UDG were added. To the other was added 0.5 µl water. Both tubes were incubated at 37° for 15 minutes, and to each was added 1 µl 1 N NaOH. The tubes were incubated at 95° for 15 minutes and 1 µl 12.5% acetic acid was added to neutralize the mixtures. About one third of each reaction was applied to a 12% sequencing gel. The presence of the 3' dU of oligonucleotide #1088 was confirmed by the UDG-specific cleavage of the LCR products. The size of the cleavage product was precisely that expected from the structures of the participating oligonucleotides. In other words, the ligation product of oligonucleotides 1088 and 1089 was cleaved by the combination of UDG and elevated pH and temperature, indicating that a uracil was present at the ligation junction of the participating oligonucleotides.

Uracil bases located at certain positions within oligodeoxynucleotides, especially at or near the 5' and 3' ends, were not removed by the enzyme uracil DNA glycosylase. This can be the basis of a method to control carryover contamination of Ligation Chain Reaction amplifications, if the uracil bases at these certain locations become sensitive to UDG when the oligodeoxynucleotide(s) become incorporated into LCR products.

In the example described here, deoxyuracil bases at the 3' ends of oligodeoxynucleotides are not recognized by UDG. Once the oligonucleotides containing the 3' deoxyuracil was joined or ligated by LCR, the ligation products became sensitive to cleavage with UDG and alkaline hydrolysis. Thus, this treatment rendered the amplification products unamplifiable in subsequent LCR reactions.

### EXAMPLE 6

A modification of LCR as described in Example 5 was performed to determine the ability of UDG alone (without alkaline hydrolysis of the sugar-phosphate backbone) to prevent amplification of uracil-containing amplification products.

20 µl reactions contained as indicated 100 fmol probe oligos (1088 and 1091 with 5' OH and 3' dU; 1089 and 1090 with 5' phosphate; 1089 labelled with ³²P); 5 fmol target #1092 (internal dU) or #1058 (no dU); 5 units Taq ligase; and 0.25 ul BRL UDG.

Reactions were assembled and incubated under mineral oil in a Cetus/Perkin-Elmer Thermal Cycler: 37°C 90 min, 85°C 5 min, then 20 cycles of 85°C 30 sec, 55°C 2 min, followed by incubation at 0°C.

1.25 ul aliquots of each reaction were applied to a 12.5% polyacrylamide/8M urea gel and electrophoresed at 70 watts power for about 90 minutes. An autoradiograph was made of the wet gel at -70°C using an intensifying screen.

In the absence of target, about 5% of the probe oligo was ligated to larger product This represents target-independent background. In the presence of 5 finol of target, about 70 fmol of probe oligo (70% of the total present in the reaction) ligated to form product. When 0.25 µl of UDG were present in the reaction containing dT target, no effect was observed on the amount of ligation product obtained (about 70 fmol). In contrast, only about 10 fmol of ligation product was obtained when UDG was incubated with dU target (mock contaminant). These observations show that the 3' dU of oligos 1088 and 1091 were not affected by the UDG, and that greater than 90% of the dU mock contaminant was converted by UDG incubation to a form that could not be a substrate for ligation of probe oligonucleotides.

UDG treatment of uracil-containing "ligation products" (#1092) were substantially prevented from being amplified during Ligase Chain Reaction. However, uracil-containing "ligation products" not treated with UDG were amplified. Thus, UDG incubation alone, without further treatment, is sufficient to substantially prevent amplification of uracil-containing ligation contaminants which may be present in subsequent LCR reaction samples.

## Claims

1. A process of oligonucleotide-dependent amplification of one or more nucleic acid sequences, the process comprising:
(a) amplifying nucleic acid present in a first sample to produce amplified first nucleic acid containing deoxyuridine;
(b) subjecting a second sample to uracil DNA glycosylase treatment, which sample may be contaminated by the amplification product of (a); wherein the second sample contains a second nucleic acid and one or more oligonucleotides specific for use in amplifying said second nucleic acid, at least one of the oligonucleotides comprising deoxyuridine at one or more of its termini; and
(c) amplifying the second nucleic acid;
said at least one of the oligonucleotides being substantially less susceptible to uracil DNA glycosylase treatment than the amplified first nucleic acids containing deoxyuridine, so that uracil containing amplification products of (a) which may contaminate the second sample are rendered unamplifiable.

2. A method of detecting target nucleic acid in a sample, the method comprising:
(a) providing nucleic acid in the sample as single-stranded nucleic acid;
(b) providing the sample with at least four DNA probes, wherein:
(1) the first probe is a single strand capable of hybridizing to a first segment of a primary strand of a target nucleic acid;
(2) the second probe is a single strand capable of hybridizing to a second segment of the primary strand of the target nucleic acid sequence;
(3) the 5' end of the first segment of the primary strand of the target is positioned relative to the 3' end of the second segment of the primary strand of the target to enable joining of the 3' end of the first probe to the 5' end of the second probe, when the probes are hybridized to the primary strand of the target nucleic acid;
(4) the third probe is capable of hybridizing to the first probe;
(5) the fourth probe is capable of hybridizing to the second probe;
(6) at least the 3' nucleotide of the first probe or the 5' nucleotide of the second probe is deoxyuridine; and
(7) at least the 3' nucleotide of the fourth probe or the 5' nucleotide of the third probe is deoxyuridine; and
(c) treating the sample with uracil DNA glycosylase; so that glycosidic bonds between the uracil and deoxyribose moieties of deoxyuridine of unjoined probes are substantially unaffected and glycosidic bonds between the uracil and deoxyribose moieties of deoxyuridine of joined probes are substantially cleaved, thereby forming an apyrimidinic deoxyribose in a sugar-phosphate backbone of a nucleic acid;
(d) repeatedly performing the cycle as follows:
(1) hybridizing the probes with nucleic acid in the sample;
(2) ligating hybridized probes to form joined probe sequences; and
(3) denaturing DNA in the sample; and
(e) detecting the joined probe sequences.

3. A method as claimed in claim 2 wherein:
in (6) both the 3' nucleotide of the first probe and the 5' nucleotide of the second probe are deoxyuridine;
in (7) both the 3' nucleotide of the fourth probe and the 5' nucleotide of the third probe are deoxyuridine; and/or
in (6) and (7) both the 3' nucleotide of the first probe and the 3' nucleotide of the fourth probe are deoxyuridine.

4. A method as claimed in claim 2 or claim 3 additionally comprising:
(f) treating the sample with endonuclease IV, and/or subjecting the sample to alkaline hydrolysis; to cleave a sugar-phosphate backbone; and
(g) optionally terminating the treatment such as by heating.

5. A method as claimed in any of claims 2 to 4 wherein;
the probes are joined by ligase;
the target nucleic acid sequences is DNA;
the joined nucleic acid is separated from the target sequence by heat denaturation; and/or
the cycle is repeated at least twice, such as between 20 and 50 times.

6. A method as claimed in any of claims 2 to 5 wherein the 5' end of the second probe, but not of the first probe, is phosphorylated, and/or the target sequence is double-stranded before (a).

7. A kit suitable for performing an assay according to the method of claim 2, the kit comprising a container comprising the probes as disclosed in claim 2, a container comprising a nucleic acid ligase, a container comprising a uracil DNA glycosylase and optionally a container comprising endonuclease IV.

8. A process as claimed in claim 1, further comprising the step of:
(d) terminating the treatment of (b).

9. A process as claimed in claim 8, wherein the terminating is by heating.

10. A process as claimed in claim 1, wherein the oligonucleotide-dependent amplification is of two separate complementary strands, of equal or unequal length, and whereby amplifying the second nucleic acid (c) comprises the following steps:
(e) treating the complementary strands with two oligonucleotide primers, for each different specific sequence being amplified, under conditions such that for each different sequence being amplified, an extension product of each primer is synthesized which is complementary to each nucleic acid strand, the primers being sufficiently complementary to different strands of each specific sequence to hybridize therewith so that the extension product synthesized from one primer, when it is separated from its complement, can serve as a template for synthesis of the extension product of the other primer;
(f) separating the primer extension products from the templates on which they were synthesized to produce single-stranded molecules; and
(g) treating the single-stranded molecules generated from step (f) with the primers of step (e) under conditions that a primer extension product is synthesized using each of the single strands produced in step (f) as a template.

## Patentansprüche

1. Verfahren zur oligonukleotidabhängigen Amplifikation einer oder mehrerer Nukleinsäuresequenzen, welches Verfahren folgendes umfaßt:
(a)Amplifizieren einer Nukleinsäure, die in einer ersten Probe vorhanden ist, zur Erzeugung einer amplifizierten ersten Nukleinsäure, die Desoxyuridin enthält;
(b)Unterziehen einer zweiten Probe einer Uracil-DNA-Glycosylasebehandlung, wobei die Probe durch das Amlifizierungsprodukt von (a) verunreinigt sein kann; wobei die zweite Probe eine zweite Nukleinsäure und ein oder mehrere Oligonukleotide enthält, die zur Verwendung in der Amplifizierung der zweiten Nukleinsäure spezifisch sind, wobei mindestens eines -der Oligonukleotide Desoxyuridin an einem oder mehreren seiner Termini umfaßt; und
(c)Amplifizieren der zweiten Nukleinsäure;
wobei das mindestens eine der Oligonukleotide im wesentlichen für eine Uracil-DNA-Glycosylasebehandlung weniger empfänglich ist als die amplifizierten ersten Nukleinsäuren, die Desoxyuridin enthalten, so daß uracilhaltige Amplifikationsprodukte von (a), welche die zweite Probe verunreinigen könnten, unamplifizierbar werden.

2. Verfahren für den Nachweis einer Targetnukleinsäure in einer Probe, welches Verfahren folgendes umfaßt:
(a)Bereitstellen einer Nukleinsäure in der Probe als einzelsträngige Nukleinsäure;
(b)Versehen der Probe mit mindestens vier DNA-Sonden, wobei:
(1) die erste Sonde ein Einzelstrang ist, der an ein erstes Segment eines primären Strangs einer Targetnukleinsäure hybridisieren kann;
(2) die zweite Sonde ein Einzelstrang ist, der an ein zweites Segment des primären Strangs der Targetnukleinsäuresequenz hybridisieren kann;
(3) das 5'-Ende des ersten Segments des primären Strangs des Targets relativ zu dem 3'-Ende des zweiten Segments des primären Strangs des Targets angeordnet ist, um eine Verbindung des 3'-Endes der ersten Sonde mit dem 5'-Ende der zweiten Sonde zu ermöglichen, wenn die Sonden an den primären Strang der Targetnukleinsäure hybridisiert werden;
(4) die dritte Sonde an die erste Sonde hybridisieren kann;
(5) die vierte Sonde an die zweite Sonde hybridisieren kann;
(6) mindestens das 3'-Nukleotid der ersten Sonde oder das 5'-Nukleotid der zweiten Sonde Desoxyuridin ist; und
(7) mindestens das 3'-Nukleotid der vierten Sonde oder das 5'-Nukleotid der dritten Sonde Desoxyuridin ist; und
(c) Behandeln der Probe mit Uracil-DNA-Glycosylase; so daß Glykosidbindungen zwischen dem Uracil und Desoxyriboseteilen von Desoxyuridin nicht verbundener Sonden im wesentlichen nicht betroffen sind und Glycosidbindungen zwischen dem Uracil und Desoxyriboseteilen von Desoxyuridin verbundener Sonden im wesentlichen gespalten sind, wodurch eine apyrimidinische Desoxyribose in einer Zucker-Phosphat-Hauptkette einer Nukleinsäure gebildet wird.
(d)wiederholte Durchführung des Zyklus wie folgt:
(1) Hybridisieren der Sonden mit Nukleinsäure in der Probe;
(2) Ligieren hybridisierter Sonden zur Bildung verbundener Sondensequenzen; und
(3) Denaturieren von DNA in der Probe; und
(e)Nachweisen der verbundenen Sondensequenzen.

3. Verfahren nach Anspruch 2, wobei:
in (6) sowohl das 3'-Nukleotid der ersten Sonde als auch das 5'-Nukleotid der zweiten Sonde Desoxyuridin sind;
in (7) sowohl das 3'-Nukleotid der vierten Sonde als auch das 5'-Nukleotid der dritten Sonde Desoxyuridin sind; und/oder
in (6) und (7) sowohl das 3'-Nukleotid der ersten Sonde als auch das 3'-Nukleotid der vierten Sonde Desoxyuridin sind.

4. Verfahren nach Anspruch 2 oder Anspruch 3, zusätzlich umfassend:
(f) Behandeln der Probe mit Endonuklease IV und/oder Unterziehen der Probe einer alkalischen Hydrolyse; zur Spaltung einer Zucker-Phosphat-Hauptkette; und
(g)wahlweises Beenden der Behandlung, wie durch Erwärmung.

5. Verfahren nach einem der Ansprüche 2 bis 4, wobei:
die Sonden durch Ligase verbunden werden;
die Targetnukleinsäuresequenzen DNA sind;
die verbundene Nukleinsäure von der Targetsequenz durch Wärmedenaturierung getrennt wird; und/oder
der Zyklus mindestens zweimal wiederholt wird, wie zum Beispiel 20 bis 50 mal.

6. Verfahren nach einem der Ansprüche 2 bis 5, wobei das 5'-Ende der zweiten Sonde, aber nicht der ersten Sonde, phosphoryliert ist und/oder die Targetsequenz vor (a) doppelsträngig ist.

7. Testsatz, der zur Durchführung eines Tests nach dem Verfahren von Anspruch 2 zweckdienlich ist, welcher Testsatz einen Behälter mit den in Anspruch 2 offenbarten Sonden, einen Behälter mit einer Nukleinsäureligase, einen Behälter mit einer Uracil-DNA-Glycosylase und wahlweise einen Behälter mit Endonuclease IV umfaßt.

8. Verfahren nach Anspruch 1, welches ferner folgenden Schritt umfaßt:
(d)Beenden der Behandlung von (b).

9. Verfahren nach Anspruch 8, wobei das Beenden durch Erwärmung erfolgt.

10. Verfahren nach Anspruch 1, wobei die oligonukleotidabhängige Amplifikation zwei getrennte komplementäre Stränge gleicher oder ungleicher Länge betrifft und wobei die Amplifizierung der zweiten Nukleinsäure (c) die folgenden Schritte umfaßt:
(e) Behandeln der komplementären Stränge mit zwei Oligonukleotidprimern, für jede unterschiedliche, spezifische, zu amplifizierende Sequenz, unter solchen Bedingungen, daß für jede unterschiedliche zu amplifizierende Sequenz ein Extensionsprodukt jedes Primers synthetisiert wird, das zu jedem Nukleinsäurestrang komplementär ist, wobei die Primer ausreichend komplementär zu verschiedenen Strängen jeder spezifischen Sequenz sind, um mit diesen zu hybridisieren, so daß das Extensionsprodukt, das von einem Primer synthetisiert wird, bei Abtrennung von seinem Komplement als Matrize zur Synthese des Extensionsprodukts des anderen Primers dienen kann;
(f) Abtrennen der Primerextensionsprodukte von den Matrizen, auf welchen sie synthetisiert wurden, um einzelsträngige Moleküle herzustellen; und
(g)Behandeln der einzelsträngigen Moleküle, die in Schritt (f) erzeugt wurden, mit den Primern von Schritt (e) unter solchen Bedingungen, daß ein Primerextensionsprodukt unter Verwendung jedes der Einzelstränge, die in Schritt (f) erzeugt wurden, als Matrize synthetisiert wird.

## Revendications

1. Procédé d'amplification, dépendante des oligonucléotides, d'une ou plusieurs séquences d'acides nucléiques, le procédé comprenant :
(a) l'amplification de l'acide nucléique présent dans un premier échantillon pour produire un premier acide nucléique amplifié contenant de la désoxyuridine;
(b) le traitement d'un deuxième échantillon par l'uracile ADN glycosylase, cet échantillon pouvant être contaminé par le produit d'amplification de (a); dans lequel le deuxième échantillon contient un deuxième acide nucléique et un ou plusieurs oligonucléotides, spécifiques de l'utilisation dans l'amplification dudit deuxième acide nucléique, l'un au moins des oligonucléotides comprenant une désoxyuridine à l'une ou plus de ses extrémités, et
(c) l'amplification du deuxième acide nucléique;
ledit au moins un des oligonucléotides étant beaucoup moins sensible au traitement par l'uracile ADN glycosylase que les premiers acides nucléiques amplifiés contenant de la désoxyuridine, de telle sorte que les produits d'amplification de (a) contenant de l'uracile, qui peuvent contaminer le deuxième échantillon, sont rendus impossibles à amplifier.

2. Procédé de détection d'un acide nucléique cible dans un échantillon, le procédé comprenant :
(a) la fourniture de l'acide nucléique dans l'échantillon sous forme d'acide nucléique simple brin;
(b) l'addition à l'échantillon d'au moins quatre sondes d'ADN, dans lesquelles :
(1) la première sonde est un simple brin capable de s'hybrider avec un premier segment d'un brin primaire d'un acide nucléique cible;
(2) la deuxième sonde est un simple brin capable de s'hybrider avec un deuxième segment du brin primaire de la séquence d'acide nucléique cible;
(3) l'extrémité 5' du premier segment du brin primaire de la cible est positionnée, par rapport à l'extrémité 3' du deuxième segment du brin primaire de la cible, pour permettre de joindre l'extrémité 3' de la première sonde à l'extrémité 5' de la deuxième sonde, lorsque les sondes sont hybridées au brin primaire de l'acide nucléique cible;
(4) la troisième sonde est capable de s'hybrider avec la première sonde;
(5) la quatrième sonde est capable de s'hybrider avec la deuxième sonde;
(6) au moins le nucléotide 3' de la première sonde ou le nucléotide 5' de la deuxième sonde est une désoxyuridine, et
(7) au moins le nucléotide 3' de la quatrième sonde ou le nucléotide 5' de la troisième sonde est une désoxyuridine, et
(c) le traitement de l'échantillon par l'uracile ADN glycosylase, de telle sorte que les liaisons glycosidiques entre l'uracile et les parties désoxyribose de la désoxyuridine des sondes non jointes ne soient dans l'ensemble pas affectées, et que les liaisons glycosidiques entre l'uracile et les parties désoxyribose de la désoxyuridine des sondes jointes soient en grande partie clivées, formant ainsi un désoxyribose non pyrimidinique dans le squelette sucre-phosphate d'un acide nucléique;
(d) la réalisation du cycle de façon répétée comme suit :
(1) hybridation des sondes avec l'acide nucléique de l'échantillon;
(2) ligature des sondes hybridées pour former des séquences de sondes jointes, et
(3) dénaturation de l'ADN de l'échantillon, et
(e) la détection des séquences des sondes jointes.

3. Procédé suivant la revendication 2, dans lequel :
en (6), le nucléotide 3' de la première sonde et le nucléotide 5' de la deuxième sonde sont tous deux des désoxyuridine;
en (7), le nucléotide 3' de la quatrième sonde et le nucléotide 5' de la troisième sonde sont tous deux des désoxyuridine, et/ou
en (6) et (7), le nucléotide 3' de la première sonde et le nucléotide 3' de la quatrième sonde sont tous deux des désoxyuridine.

4. Procédé suivant la revendication 2 ou 3, comprenant de plus :
(f) le traitement de l'échantillon par l'endonucléase IV, et/ou l'exposition de l'échantillon à une hydrolyse alcaline, pour cliver un squelette sucre-phosphate, et
(g) l'arrêt facultatif du traitement, par exemple par chauffage.

5. Procédé suivant l'une quelconque des revendications 2 à 4, dans lequel :
les sondes sont jointes par une ligase;
les séquences cibles d'acide nucléique sont de l'ADN;
l'acide nucléique joint est séparé de la séquence cible par dénaturation thermique, et/ou le cycle est répété au moins deux fois, par exemple entre 20 et 50 fois.

6. Procédé suivant l'une quelconque des revendications 2 à 5, dans lequel l'extrémité 5' de la deuxième sonde, mais pas de la première sonde, est phosphorylée, et/ou où, avant (a), la séquence cible est en double brin.

7. Kit convenant à la réalisation d'un dosage suivant le procédé de la revendication 2, le kit comprenant un récipient comprenant les sondes comme décrit dans la revendication 2, un récipient comprenant une ligase pour acides nucléiques, un récipient comprenant une uracile ADN glycosylase et, facultativement, un récipient comprenant de l'endonucléase IV.

8. Procédé suivant la revendication 1, comprenant de plus l'étape de :
(d) l'arrêt du traitement de (b).

9. Procédé suivant la revendication 8, dans lequel l'arrêt se fait par chauffage.

10. Procédé suivant la revendication 1, dans lequel l'amplification dépendant des oligonucléotides est de deux brins complémentaires séparés, de longueurs égales ou inégales, et dans lequel l'amplification (c) du second acide nucléique comprend les étapes suivantes :
(e) le traitement des brins complémentaires avec deux amorces d'oligonucléotides, pour chaque séquence spécifique différente à amplifier, dans des conditions telles que, pour chaque séquence différente à amplifier, un produit d'extension de chaque amorce, complémentaire de chaque brin d'acide nucléique, est synthétisée, les amorces étant suffisamment complémentaires de différents brins de chaque séquence spécifique pour s'y hybrider, de telle sorte que le produit d'extension synthétisé à partir d'une amorce, lorsqu'il est séparé de son complément, peut servir de matrice pour la synthèse du produit d'extension de l'autre amorce;
(f) la séparation des produits de l'extension de l'amorce des matrices sur lesquelles ils sont synthétisés, pour produire des molécules simple brin, et
(g) le traitement des molécules simple brin générées dans l'étape (f), avec les amorces de l'étape (e), dans des conditions où un produit d'extension de l'amorce est synthétisé, en utilisant comme matrice chacun des simples brins produits dans l'étape (f).
